# EUROPÄISCHE PATENTSCHRIFT

(11) **EP 3 518 757 B1**
(45) Veröffentlichungstag und Bekanntmachung des Hinweises auf die Patenterteilung: **04.12.2024**
(21) Anmeldenummer: 17777858.6
(22) Anmeldetag: 26.09.2017
(51) Int. Cl.: A61B 5/0245, A61B 5/11, A61B 5/316, A61B 5/352, A61B 5/366, A61B 5/00, G16H 40/63, G16H 50/20, G16H 50/70

(54) **ÜBERWACHUNG VON BIOSIGNALEN, INSBESONDERE ELEKTROKARDIOGRAMMEN**
MONITORING OF BIOSIGNALS, IN PARTICULAR ELECTROCARDIOGRAMS
SURVEILLANCE DE SIGNAUX BIOLOGIQUES, EN PARTICULIER D'ÉLECTROCARDIOGRAMMES

(30) Priorität: 28.09.2016 DE 102016011700
(43) Veröffentlichungstag der Anmeldung: 07.08.2019
(73) Patentinhaber: Personal Medsystems GmbH, 60320 Frankfurt (DE)
(72) Erfinder: RIEMENSCHNEIDER, Markus, 65510 Limburg (DE); SAUERZAPF, Jürgen, 55124 Mainz (DE)
(74) Vertreter: Samson & Partner Patentanwälte mbB
(86) Internationale Anmeldenummer: PCT/EP2017/074291
(87) Internationale Veröffentlichungsnummer: WO 2018/060162

(56) Entgegenhaltungen:
- WO-A1-2016/014194
- WO-A1-2016/086037
- WO-A1-2016/110804
- WO-A2-03/057025
- WO-A2-2004/032715
- US-A1- 2004 230 105
- US-A1- 2006 259 329
- US-A1- 2007 010 753
- US-A1- 2007 142 737

## Beschreibung

### Gebiet der Erfindung

Die vorliegende Erfindung bezieht sich im Allgemeinen auf Verfahren, Systeme und Computer-Programm-Produkte zur Überwachung von Biosignalen, insbesondere Elektrokardiogrammen, sowie auf die Verwendung eines Elektrokardiogramms als Vergleichs-Elektrokardiogramm.

### Hintergrund der Erfindung

Biosignale, wie beispielsweise Elektrokardiogramme, erlauben Rückschlüsse auf die physiologischen Aktivitäten von Organen oder eines Organismus. Die Auswertung von überwachten Biosignalen wird nicht nur von Ärzten als Diagnostik-Grundlage, sondern zunehmend auch im Bereich des sogenannten Home Monitoring oder Personal Tracking vorgenommen. Hierbei überwachen Benutzer ihre körperliche Aktivität oder physiologische Messgrößen ohne die permanente Unterstützung eines Arztes. Die Überwachung kann die Grundlage bilden für eine Handlungsempfehlung an den Benutzer, beispielsweise die Anweisung, einen Arzt für eine eingehende Diagnostik aufzusuchen. Ferner kann die Überwachung zur Auswertung oder Steuerung eines sportlichen Trainingsplans, z.B. zur Ausdauersteigerung im Marathon-Training, genutzt werden.

Die Auffälligkeit oder Unauffälligkeit von überwachten Biosignalen kann durch Vergleich mit einer Referenz bestimmt werden. Hierbei kann die Referenz vorbestimmt sein durch Messung an einem anderen Probanden, durch Mittelwertbildung über eine Gruppe von Probanden oder durch computer-gestützte Modellierung der zugrundeliegenden physiologischen und/oder pathologischen Vorgänge.

Die Zuverlässigkeit des Vergleichs (bestimmt durch die Anteile der falsch-positiven und/oder falsch-negativen Vergleichsergebnisse) hängt hierbei unter anderem von der Qualität der Referenz ab. Ferner kann die Zuverlässigkeit des Vergleichs erhöht werden, wenn Referenz und Kontrollmessung sich auf vergleichbare Bedingungen beziehen. Diese Bedingungen umfassen die körperlichen Gegebenheiten des Probanden und Umwelteinflüsse.

Aus der WO 96/25096 ist bekannt, Muster-Elektrokardiogramme zu bestimmten kardialen Ereignissen bekannter Diagnose, z.B. Tachykardie oder Vorhofflimmern, zu speichern und mit einem gemessenen Elektrokardiogramm zu vergleichen für eine genauere Lokalisierung von Störungen.

Aus der WO 2016/014194 A1 sind Verfahren und Vorrichtungen zur Unterscheidung einer supraventrikulären Tachykardie von einer ventrikulären Tachykardie bei Änderungen der Körperhaltung bekannt.

Aus der US 2007/142737 A1 ist bekannt, bei der Unterscheidung einer supraventrikulären Tachykardie von einer ventrikulären Tachykardie die Herzratenabhängigkeit zu berücksichtigen.

Die WO 2016/086037 A1 offenbart ein Verfahren mit den Merkmalen des Oberbegriffs von Anspruch 1.

Vor diesem Hintergrund ist es Aufgabe der vorliegenden Erfindung, verbesserte Verfahren, Systeme und Computer-Programm-Produkte zur Überwachung von Biosignalen, insbesondere Elektrokardiogrammen, bereitzustellen. Hierbei bezieht sich die Erfindung auf jene Fälle, bei denen die Bestimmung der Messbedingungen keine Diagnose eines pathologischen, insbesondere eines kardialen, Ereignisses voraussetzt.

### Zusammenfassung der Erfindung

Die vorliegende Erfindung stellt Verfahren, Systeme und Computer-Programm-Produkte zur Überwachung von Biosignalen, insbesondere Elektrokardiogrammen, gemäß den unabhängigen Ansprüchen zur Verfügung. Die abhängigen Ansprüche betreffen bevorzugte Ausführungsformen.

In einem ersten Aspekt stellt die vorliegende Erfindung ein Verfahren zur Überwachung von Biosignalen eines Probanden bereit, wie es in Anspruch 1 definiert wird.

In manchen Ausführungsformen kann ein Biosignal in einer Messkurve, z.B. einem zeitlichen Verlauf einer gemessenen Spannung, bestehen. Bei einem Elektrokardiogramm als Beispiel einer Messkurve wird der zeitliche Verlauf der elektrischen Muskelaktivität des Herzen in Form elektrischer Potentialdifferenzen gemessen.

In manchen Ausführungsformen können die Biosignale gemessene Werte, z.B. Spannungswerte, oder aus Messungen abgeleitete Werte umfassen. Ein Beispiel von Biosignalen, die aus einer Messung abgeleitet werden können, sind die dem Fachmann geläufigen EKG-Parameter, wie QT-Zeitintervall, Zeiten im QRS-Komplex, ST-Strecke, Maße der P-Welle / R-Zacke / T-Welle, die aus einem gemessenen Elektrokardiogramm ermittelt werden können.

Gemäß der Erfindung liegen die mehreren Referenz-Biosignale kontinuierlich als mehrere Intervalle einer Zeitreihe vor. In von den Ansprüchen nicht umfassten und lediglich zu Illustrationszwecken dargestellten Beispielen können die mehreren Referenz-Biosignale diskret, z.B. in Form von Datenbankeinträgen und/oder einer oder mehrerer Datei(en) und/oder einer Ordnerstruktur, vorliegen.

Unter einer kontinuierlichen Zeitreihe, beispielsweise von Herzfrequenzwerten, sollen hier auch quantisierte quasi-kontinuierliche Zeitreihen verstanden werden. Herzfrequenzen können typischerweise mit einem Abstand von 1 Herzschlag gemessen werden, womit zwischen zwei Messpunkten ein gewisser zeitlicher Abstand liegt. Nichtsdestotrotz können diese quasikontinuierlichen Zeitreihen für die Zwecke der vorliegenden Erfindung und für Illustrationszwecke als kontinuierlich angesehen werden. In manchen Ausführungsbeispielen kann eine quantisierte Messreihe mittels Interpolationsverfahren in eine im strikten Sinne kontinuierliche Zeitreihe überführt werden.

Im Allgemeinen wird jedem der mehreren Referenz-Biosignale ein jeweiliger Wert eines Referenzparameters zugeordnet. Werte des Referenzparameters beschreiben zumindest teilweise physiologische Zustände des Probanden. Insbesondere können Referenzparameter Einfluss auf Biosignale haben. Im Allgemeinen setzen Referenzparameter (oder eine Bestimmung eines Werts eines Referenzparameters) keine Diagnose eines pathologischen Ereignisses, insbesondere eines kardialen Ereignisses, voraus. Beispielsweise soll erfindungsgemäß nicht das Vorliegen oder Nichtvorliegen einer pathologischen Diagnose (z.B. einer Herzrhythmusstörung) als Referenzparameter zur Auswahl eines Vergleichs-Biosignals (z.B. eines Vergleichs-Elektrokardiogramms) herangezogen werden.

In manchen Ausführungsformen kann der Referenzparameter numerische Werte annehmen, gegebenenfalls in Verbindung mit einer Einheit. Der Referenzparameter umfasst die Herzfrequenz, welche in der Einheit "Schläge pro Minute" angeben sein kann. Der Zahlenwert kann je nach Referenzparameter ganzzahlige und/oder Fließkomma-Werte annehmen. In manchen Ausführungsformen kann der Referenzparameter beschreibende Werte annehmen. Ein Beispiel eines Referenzparameters mit beschreibenden Werten ist die Körperhaltung, mit Werten wie "liegend", "sitzend", "stehend".

Beispiele von Referenzparametern, deren Werte physiologische Zustände des Probanden zumindest teilweise beschreiben, sind:
- Herzfrequenz, aus einem Elektrokardiogramm ermittelbare EKG-Parameter (z.B. QT-Zeitintervall, Zeiten und/oder Amplituden im QRS-Komplex, ST-Strecke, P-Welle, R-Zacke, T-Welle, Q-Zacke, S-Zacke, U-Welle, Nulllinie), Blutzuckerspiegel, Blutdruck, Sauerstoffgehalt im Blut (SpO2) , Elektrolyt-Spiegel (z.B. Konzentration von Calcium, Kalium, Natrium), Körpertemperatur, Atemfrequenz, Atemminutenvolumen;

Diese Beispiele von Referenzparametern können durch numerische Werte, gegebenenfalls in Verbindung mit einer jeweils passenden Einheit, angegeben werden.

Weitere Beispiele von Referenzparametern, deren Werte physiologische Zustände des Probanden zumindest teilweise beschreiben, sind:
- Medikation des Probanden; Körperhaltung des Probanden, aktueller und/oder bisheriger Bewegungszustand des Probanden; Lebensgewohnheiten des Probanden, insbesondere dessen Berufstätigkeit, Essgewohnheiten, Häufigkeit sportlicher Betätigung.

Diese weiteren Beispiele von Referenzparametern können durch beschreibende Werte, gegebenenfalls numerisch oder binär kodiert, angegeben werden. So kann der Referenzparameter "Medikation des Probanden" beispielsweise Werte wie "ja", "nein", "Amiodaron ja", "Amiodaron nein" oder eine Vielzahl anderer je nach Proband und/oder je nach zu überwachendem Biosignal zu bestimmende Werte annehmen. Der Referenzparameter "Bewegungszustand" kann beschreibende Werte, wie beispielweise "Ruhe"; "sportliche Betätigung", "laufend", "bis soeben gelaufen"; "Belastung auf dem Ergometer", etc. annehmen.

Werte von Referenzparametern können ferner zumindest teilweise Randbedingungen einer Umgebung beschreiben. Beispiele von Referenzparametern, deren Werte Randbedingungen der Umwelt des Probanden zumindest teilweise beschreiben sind:
- Tageszeit, Umgebungstemperatur, Luftdruck, Luftfeuchtigkeit, Jahreszeit.

Die genannten Beispiele von Referenzparametern sollen hier Illustrationszwecken dienen und nicht als eine beschränkende Aufzählung verstanden werden. Andere Referenzparameter als die genannten sind ebenso umfasst, solange sie Einfluss auf Biosignale haben können. Gemäß der Erfindung umfasst der Referenzparameter jedenfalls eine Herzfrequenz.

In manchen Ausführungsformen kann der Referenzparameter mehrere Komponenten umfassen. In solchen Fällen kann man von einem mehr-komponentigen Referenzparameter sprechen oder von einem Referenzparameter, der aus einem Satz mehrere Komponenten besteht. Beispielsweise können die Komponenten "Herzfrequenz" und "Körperhaltung" den Satz "(Herzfrequenz; Körperhaltung)" als ein Referenzparameter bilden. Werte eines mehr-komponentigen Referenzparameters sind durch die Werte seiner Komponenten bestimmt. So kann beispielsweise "(62 Schläge pro Minute; liegend)" ein möglicher Wert des Referenzparameters "(Herzfrequenz; Körperhaltung)" sein, wobei "62 Schläge pro Minute" ein möglicher Wert der Komponente "Herzfrequenz" ist und "liegend" ein möglicher Wert der Komponente "Körperhaltung" ist. Ein Satz von n Komponenten kann mathematisch als n-Tupel dargestellt werden, d.h. derart dargestellt werden, dass die Reihenfolge der Komponenten nicht vernachlässigbar ist. In manchen Ausfiihrungsformen können die Komponenten jeweils unterschiedlich gewichtet sein.

Das Verfahren umfasst ferner das Messen eines Kontroll-Biosignals, insbesondere eines Kontroll-Elektrokardiogramms, des Probanden, sowie das Bestimmen eines Werts des Referenzparameters, der dem Kontroll-Biosignal zugeordnet wird. Das Bestimmen kann eine Messung, eine automatische Erkennung oder eine Eingabe durch den Nutzer umfassen.

Bevorzugt erfolgt die Zuordnung des Kontroll-Biosignals und des bestimmten Werts aufgrund einer zeitlichen Nähe oder gar Simultaneität von Messung des Kontroll-Biosignals und Bestimmung des Werts des Referenzparameters.

Das Verfahren umfasst ferner das Auswählen mindestens eines Vergleichs-Biosignals, insbesondere mindestens eines Vergleichs-Elektrokardiogramms, aus den mehreren gespeicherten Referenz-Biosignalen, basierend auf dem Wert des Referenzparameters, der dem Kontroll-Biosignal zugeordnet ist, und auf den mehreren Werten des Referenzparameters, die den mehreren Referenz-Biosignalen zugeordnet sind. Bevorzugt wird ein einziges Vergleichs-Biosignal ausgewählt.

In manchen Ausführungsformen wird dasjenige der mehreren Referenz-Biosignale als Vergleichs-Biosignal ausgewählt, dessen zugeordneter Wert des Referenzparameters mit dem dem Kontroll-Biosignal zugeordneten Wert des Referenzparameters identisch ist oder die geringste Differenz aufweist.

In manchen Ausführungsformen wird dasjenige der mehreren Referenz-Biosignale als Vergleichs-Biosignal ausgewählt, dessen zugeordneter Wert des Referenzparameters den nächsthöheren oder den nächstniedrigeren Wert zu dem dem Kontroll-Biosignal zugeordneten Wert des Referenzparameters darstellt.

In manchen Ausführungsformen, insbesondere im Fall von mehr-komponentigen Referenzparametern kann eine Norm zum Zwecke des Vergleichens der mehr-komponentigen Werte des Referenzparameters definiert sein. Als illustratives Beispiel sei hier der Satz "(Herzfrequenz; Blutzuckerspiegel)" als mehr-komponentiger Referenzparameter genannt: Es kann sich die Frage stellen, ob ein Kontrollwert von (65 bpm; 105 mg/dl) "näher an" (60 bpm; 90 mg/dl) oder an (70 bpm; 110 mg/dl) liegt. Zur Regelung dieser Frage kann eine Norm zu dem jeweiligen mehr-komponentigen Satz gebildet werden, beispielsweise eine euklidische Norm (gewichtet und/oder unter Berücksichtigung der Einheiten) oder eine konditionelle Norm. Eine konditionelle Norm kann beispielsweise vorschreiben, dass zunächst ausschließlich der erstgenannte Parameter (im Beispiel: Herzfrequenz) betrachtet werden soll und nur im Falle eines uneindeutigen Ergebnisses (wie im Beispiel identische Differenz von 5 bpm) der zweitgenannte Parameter (im Beispiel: Blutzuckerspiegel) zurate gezogen werden soll. Im genannten Beispiel würde im Falle dieser konditionellen Norm der Wert (70 bpm; 110 mg/dl) ausgewählt werden. Als alternative Norm kann eine euklidische Norm im genannten Fall die Zahlenwerte von Herzfrequenz in der Einheit bpm und von Blutzuckerspiegel in der Einheit mg/dl in einem (gegebenenfalls gewichteten) euklidischen Koordinatensystem darstellen und die Differenz als Länge des Differenzvektors definieren.

Im Allgemeinen umfasst das Verfahren ferner das Vergleichen des gemessenen Kontroll-Biosignals mit dem ausgewählten Vergleichs-Biosignal. Das Vergleichen der Biosignale kann zusätzlich oder alternativ insbesondere das Vergleichen von aus den zu vergleichenden Biosignalen abgeleiteten Werten umfassen. So können beispielsweise zum Vergleich zweier Elektrokardiogramme einer oder mehrere der geläufigen EKG-Parameter (Amplituden, zeitlicher Verlauf, Rhythmusparameter, QT-Zeitintervall, Zeiten im QRS-Komplex, ST-Strecke, Maße der P-Welle / R-Zacke / T-Welle) verglichen werden.

Im Allgemeinen können die Referenz-Biosignale, beispielsweise zum Zeitpunkt ihrer Erfassung, Messung oder Abfrage als unauffällig oder als auffällig anzusehen sein. In manchen Ausführungsbeispielen können beispielsweise Referenz-Biosignale, die an einem gesunden Probanden aufgenommen wurden, als unauffällig anzusehen sein. Bei einem als unauffällig eingestuften Referenz-Biosignal kann ein negatives Vergleichsergebnis (d.h. Nicht-Übereinstimmung von Kontroll- und Referenz-Signal) zu der Handlungsanweisung, einen Arzt aufzusuchen, führen. Bei einem als auffällig eingestuften Referenz-Biosignal kann ein positives Vergleichsergebnis (d.h. Übereinstimmung von Kontroll- und Referenz-Signal) zu der Handlungsanweisung, einen Arzt aufzusuchen, führen.

In manchen Ausführungsformen können bestimmte Anforderungen an die Datenqualität der Referenz-Biosignale, beispielsweise ein hohes Signal/Rausch-Verhältnis, gestellt sein. Biosignale, die solche Anforderungen erfüllen, können als für einen Vergleich geeignet angesehen werden.

In manchen Ausführungsformen kann das gemessene Kontroll-Biosignal in die Menge der mehreren Referenz-Biosignale aufgenommen werden. Der dem gemessenen Kontroll-Biosignal zugeordnete Wert des Referenzparameters kann dem neu aufgenommenen Referenz-Biosignal zugeordnet werden. Somit kann die Menge der Referenz-Biosignale im Betrieb für künftige Verfahrensausführungen weiter vergrößert werden, sowie ein breiteres oder feiner aufgelöstes Spektrum von Werten des Referenzparameters abgedeckt werden. Eine solche Aufnahme kann insbesondere vorteilhaft sein, falls das gemessene Kontroll-Biosignal als unauffällig anzusehen ist.

In manchen Ausführungsformen kann das Verfahren ferner ein Ausgeben oder Darstellen einer Information umfassen. Beispiele von auszugebenden Informationen sind: der Wert des Referenzparameters, der dem Kontroll-Biosignal zugeordnet ist; der Wert des Referenzparameters, der dem ausgewählten Vergleichs-Biosignal zugeordnet ist; das Kontroll-Biosignal; das Vergleichs-Biosignal; eine Abweichung zwischen Kontroll-Biosignal und Vergleichs-Biosignal; eine Abweichung zwischen dem Wert des Referenzparameters, der dem Kontroll-Biosignal zugeordnet ist, und dem Wert des Referenzparameters, der dem ausgewählten Vergleichs-Biosignal zugeordnet ist; eine Handlungsempfehlung basierend auf einer Abweichung zwischen Kontroll-Biosignal und Vergleichs-Biosignal. Eine auszugebende Handlungsempfehlung kann beispielsweise auf das Hinsetzen, das Ausruhen oder die Aufnahme einer sportlichen Betätigung des Probanden gerichtet sein.

Das Ausgeben kann beispielsweise optisch, akustisch, haptisch oder in einer Kombination der genannten erfolgen.

Zu überwachende Biosignale können im Allgemeinen Rückschlüsse auf Aktivitäten von Organen oder des Organismus des Probanden ermöglichen. Eine etwaige Stellung einer Diagnose, d.h. Bestimmung einer Krankheit durch einen Arzt, basierend auf den überwachten Biosignalen ist nicht Bestandteil des erfindungsgemäßen Verfahrens.

Beispiele von zu überwachenden Biosignalen sind Blutdruckkurven, Blutbilder, Elektrokardiogramme, Elektroenzephalogramme, Elektromyogramme, Elektroretinogramme. Alternativ oder zusätzlich können Aufzeichnungen der Zusammensetzung von Atemgas (z.B. mittels Kapnometrie ermittelter CO2-Anteil) oder von Ausscheidungen (z.B. mittels Urinteststreifen ermittelte Harnzusammensetzung) als Biosignale dienen.

Die Messung von Biosignalen kann beispielsweise mittels geeigneter Sensoren, insbesondere elektrisch, wie im Falle von Elektrokardiogrammen, erfolgen. Bevorzugt erfolgt die Messung nicht-invasiv.

In manchen Ausführungsformen können mehrere Kontroll-Biosignale, insbesondere mehrere Kontroll-Elektrokardiogramme, gemessen werden. Mehrere Kontroll-Biosignale können beispielsweise als Zeitreihe gemessen werden. Dies kann insbesondere bei Langzeit-Überwachungen, wie einem Langzeit-EKG, bevorzugt sein.

Insbesondere kann zu den mehreren Kontroll-Biosignalen ein einziger Wert des Referenzparameters bestimmt werden, beispielsweise weil der Referenzparameter sich nicht wesentlich im Laufe der Zeitreihe verändert. In diesen Fällen wird aus den mehreren Referenz-Biosignalen ein einziges als Vergleichs-Biosignal ausgewählt und mit jedem der mehreren Kontroll-Biosignale verglichen.

Alternativ kann zu jedem der mehreren Kontroll-Biosignale ein jeweiliger Wert des Referenzparameters bestimmt werden, beispielsweise weil der Wert des Referenzparameters sich im Laufe der Zeitreihe verändert. In diesen Fällen wird aus den mehreren Referenz-Biosignalen zu jedem Kontroll-Biosignal ein jeweiliges Vergleichs-Biosignal ausgewählt und mit dem jeweiligen Kontroll-Biosignal verglichen.

In einem zweiten Aspekt stellt die vorliegende Erfindung ein Computerprogramm-Produkt bereit, wie es in Anspruch 11 definiert wird.

In einem dritten Aspekt stellt die vorliegende Erfindung ein System zur Überwachung von Biosignalen eines Probanden bereit, wie es in Anspruch 12 definiert wird.

Der mindestens eine Sensor ist dazu ausgelegt, ein Kontroll-Biosignal des Probanden zu messen. Insbesondere kann es sich bei dem mindestens einen Sensor um EKG-Elektroden zur Messung von Elektrokardiogrammen handeln. In manchen Ausführungsformen kann der mindestens eine Sensor kabelgebunden (z.B. per USB-Schnittstelle) oder kabellos (z.B. per Bluetooth-Schnittstelle) mit Komponenten des Systems, insbesondere mit der Steuerung, verbunden sein oder dazu ausgelegt sein, verbunden zu werden. In manchen Ausführungsformen kann der mindestens eine Sensor in oder an einem gemeinsamen Gehäuse mit zumindest der Steuerung angeordnet sein.

In manchen Ausführungsformen kann der mindestens eine Sensor ferner dazu ausgelegt sein, mehrere Referenz-Biosignale des Probanden zu messen.

Der Speicher ist dazu ausgelegt, mehrere Referenz-Biosignale, insbesondere Referenz-Elektrokardiogramme, des Probanden zu speichern. Jedem der mehreren Referenz-Biosignalen ist ein jeweiliger Wert eines Referenzparameters zugeordnet. Die zugeordneten Werte des Referenzparameters können ebenfalls in dem Speicher gespeichert sein. In manchen Ausführungsbeispielen kann der Speicher räumlich entfernt, beispielsweise auf einem Server oder in einem dezentralen Netzwerk, angeordnet sein.

Die Steuerung ist dazu eingerichtet, einen dem Kontroll-Biosignal zugeordneten Wert des Referenzparameters zu bestimmen. Bevorzugt kann die Bestimmung dieses Werts automatisch, beispielweise per Messung durch den mindestens einen Sensor oder durch einen anderen Sensor, oder manuell, beispielsweise per Nutzereingabe in einer Eingabeschnittstelle, erfolgen.

Ferner ist die Steuerung dazu eingerichtet, mindestens ein Vergleichs-Biosignal, insbesondere mindestens ein Vergleichs-Elektrokardiogramm, aus den mehreren gespeicherten Referenz-Biosignalen auszuwählen, basierend auf dem bestimmten Wert des Referenzparameters und auf den mehreren Werten des Referenzparameters, die den mehreren Referenz-Biosignalen zugeordnet sind.

Ferner ist die Steuerung dazu eingerichtet, das Kontroll-Biosignal und das Vergleichs-Biosignal zu vergleichen.

### Kurzbeschreibung der Zeichnungen

Ausführungsbeispiele der vorliegenden Erfindung werden nun beispielhaft und unter Bezugnahme auf folgende Figuren beschrieben.
Fig. 1A zeigt einen menschlichen Torso mit Sensoren zum Messen von Elektrokardiogrammen.
Fig. 1B ist eine schematische Darstellung von zwei gemessenen Elektrokardiogrammen.
Fig. 2 veranschaulicht drei Referenz-Elektrokardiogramme mit zugeordneten Werten des Referenzparameters "Herzfrequenz", sowie ein Kontroll-Elektrokardiogramm mit zugeordnetem Wert des Referenzparameters "Herzfrequenz" gemäß einem Ausführungsbeispiel.
Fig. 3A veranschaulicht drei Referenz-Elektrokardiogramme mit zugeordneten Werten des Referenzparameters "Herzfrequenz" gemäß einem weiteren Ausführungsbeispiel.
Fig. 3B veranschaulicht ein Kontroll-Elektrokardiogramm mit zugeordnetem Wert des Referenzparameters "Herzfrequenz" gemäß dem Ausführungsbeispiel von Fig. 3A.
Fig. 4 veranschaulicht drei Referenz-Elektrokardiogramme sowie ein Kontroll-Elektrokardiogramm mit jeweils zugeordneten Werten des Referenzparameters "Körperhaltung".
Fig. 5 veranschaulicht drei Referenz-Elektrokardiogramme sowie ein Kontroll-Elektrokardiogramm mit jeweils zugeordneten Werten des Referenzparameters "Umgebungstemperatur".
Fig. 6 zeigt ein Ausführungsbeispiel eines Systems gemäß der vorliegenden Erfindung,
Fig. 7 zeigt ein Ablaufdiagramm eines Verfahrens gemäß der vorliegenden Erfindung.

### Beschreibung von bevorzugten Ausführungsbeispielen

Die folgenden Ausführungsbeispiele behandeln beispielhaft Fälle von Biosignalen in Form von Elektrokardiogrammen. Elektrokardiogramme bilden elektrische Aktivitäten des Herzens in Form von zeitlichen Spannungsverläufen ab, welche beispielsweise mit Hilfe von EKG-Elektroden am Körper des Probanden gemessen werden können.

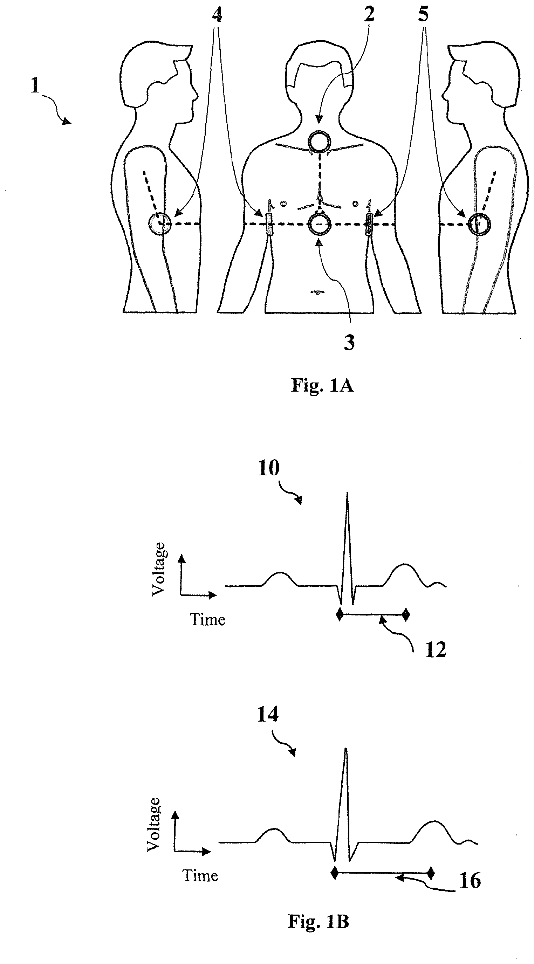

**Fig. 1A** zeigt drei Ansichten eines menschlichen Torsos 1 eines Probanden mit vier EKG-Elektroden 2-5, wovon je eine am oberen Ende des Brustbeins (Elektrode 2), am unteren Ende des Brustbeins (Elektrode 3), an der rechten mittleren Achsellinie in Höhe des unteren Brustbeinrandes (entsprechend der Höhe der Elektrode 3) (Elektrode 4) und der linken mittleren Achsellinie in Höhe des unteren Brustbeinrandes (entsprechend der Höhe der Elektrode 3) (Elektrode 5) angebracht sind. Andere Sensoranordnungen zur Messung von Elektrokardiogrammen mit einer anderen Anzahl von EKG-Elektroden, beispielsweise zehn Elektroden, sind bekannt und können ebenfalls für die Zwecke der vorliegenden Erfindung, insbesondere zur Messung von Elektrokardiogrammen, verwendet werden. Die Elektroden 2-5 können beispielsweise als Einmalelektroden zum Kleben ausgestaltet sein und ein Nassgel oder Trockengel umfassen.

Je nach Anordnung der Elektroden werden die vorhandenen Elektroden derart verschaltet, dass gewisse Ableitungen oder Kanäle erhalten werden. Die vier in Fig. 1A dargestellten Elektroden 2-5 ermöglichen, beispielsweise gemäß dem EASI-Verfahren, ein 12-Kanal-EKG zu errechnen, das mit den Ableitungen nach Einthoven (I, II, III), Goldberger (aVR, aVL, aVF) und Wilson (V1-V6), aufgezeichnet wird. Alternativ oder zusätzlich lassen sich die erweiterten Brustwandableitungen V7-V9 sowie VR3-VR9 und/oder ein EKG-Mapping so errechnen. Andere Verschaltungsschemata und/oder Ableitungen (z.B. jene nach Einthoven, Goldberger, Wilson, Nehb, Franck oder Dower) sind ebenfalls geeignet, Elektrokardiogramme für die Zwecke der vorliegenden Erfindung zu messen.

Zu Illustrationszwecken wird im Laufe der Beschreibung auf ein 12-Kanal-EKG verwiesen, welches beispielsweise mit Hilfe der vier Elektroden 2-5 aufgenommen wurde. Die vorliegende Lehre kann jedoch ebenso mit anderen als 12-Kanal-EKGs, insbesondere auch mit Vektor-EKGs oder mit 1-Kanal-EKGs, oder mit anderen Biosignalen umgesetzt werden.

**Fig. 1B** ist eine schematische Darstellung von zwei gemessenen Elektrokardiogrammen 10, 14 eines Probanden. Jedes Elektrokardiogramm ist hier dargestellt als zeitlicher Verlauf einer elektrischen Spannung über in etwa die Dauer eines Herzschlags. Bei den dargestellten Elektrokardiogrammen 10, 14 handelt es sich um die Ableitung nach Einthoven II.

Für den Zweck der vorliegenden Erfindung kann der Begriff "Elektrokardiogramm" einen einzelnen zeitlichen Verlauf für eine einzelne Ableitung, z.B. eine Ableitung nach Einthoven II, bezeichnen oder er kann eine Mehrzahl von zeitlichen Verläufen zu verschiedenen Ableitungen bezeichnen. So kann insbesondere auch die Gesamtheit der zwölf Graphen eines 12-Kanal-EKGs als "Elektrokardiogramm" bezeichnet werden.

Zu Illustrationszwecken wird im Folgenden jedes Elektrokardiogramm durch einen Graphen, z.B. die Ableitung nach Einthoven 11, dargestellt werden.

Das Elektrokardiogramm 10 ist eine schematische Darstellung eines physiologischen Elektrokardiogramms des Probanden im gesunden Zustand. Das Elektrokardiogramm wurde im Ruhezustand des Probanden als sog. Ruhe-EKG aufgenommen. Es kann als Referenzelektrokardiogramm 10 bezeichnet werden.

Aus dem gemessenen Elektrokardiogramm können die geläufigen EKG-Parameter, wie QT-Zeitintervall, Zeiten im QRS-Komplex, ST-Strecke, Maße der P-Welle, R-Zacke, T-Welle, ermittelt werden. Im Folgenden wird als Beispiel auf das QT-Zeitintervall, d.h. den zeitlichen Abstand zwischen der Q-Zacke (dem ersten negativen Ausschlag im QRS-Komplex, der durch die Kammererregung hervorgerufen wird) und der T-Welle (Erregungsrückbildung gegen Ende des EKG-Verlaufs), eingegangen. Das QT-Zeitintervall des Elektrokardiogramms 10 ist schematisch durch die Dauer 12 hervorgehoben.

Das ebenfalls in Fig. 1B dargestellte Elektrokardiogramm 14 wurde zu einem späteren Zeitpunkt als Elektrokardiogramm 10 aufgezeichnet. Es wurde am gleichen Probanden wie Elektrokardiogramm 10 aufgezeichnet. Insbesondere kann es der Überwachung oder Kontrolle der Herzaktivität im Vergleich zu dem als unauffällig eingestuften Referenzelektrokardiogramm 10 aufgezeichnet worden sein. Das später aufgezeichnete Elektrokardiogramm 14 wird als Kontroll-Elektrokardiogramm 14 bezeichnet. Das Kontroll-Elektrokardiogramm 14 weist hier ein gegenüber dem Referenzelektrokardiogramm 10 erhöhtes QT-Zeitintervall 16 auf. Das Kontroll-Elektrokardiogramm 14 kann daher als auffällig gegenüber dem Referenzelektrokardiogramm 10 anzusehen sein.

Eine Veränderung des QT-Zeitintervalls kann eine Vielzahl von Ursachen haben. So kann beispielsweise eine Erhöhung der Herzfrequenz zu einer Verringerung der QT-Zeit führen. Medikation, z.B. die Gabe von Amiodaron, kann ebenfalls zu einer Verlängerung der QT-Zeit führen. Auch besteht ein Zusammenhang zwischen bestimmten Elektrolyt-Konzentrationen im Blut und Veränderungen der QT-Zeit. Beispielsweise können eine Hypercalciämie oder eine Hyperkaliämie mit Verringerungen der QT-Zeit einhergehen. Ferner kann eine lange QT-Zeit jedoch auch ein Indikator für bestimmte Herzrythmusstörungen, wie eine Bradykardie, sein.

Ausgehend von einem Vergleich des Referenzelektrokardiogramms 10 und des Kontrollkardiogramms 14 besteht daher Ungewissheit, auf welchen der genannten Einflussfaktoren die im Vergleich festgestellte Veränderung des QT-Zeitintervalls zurückgeht.

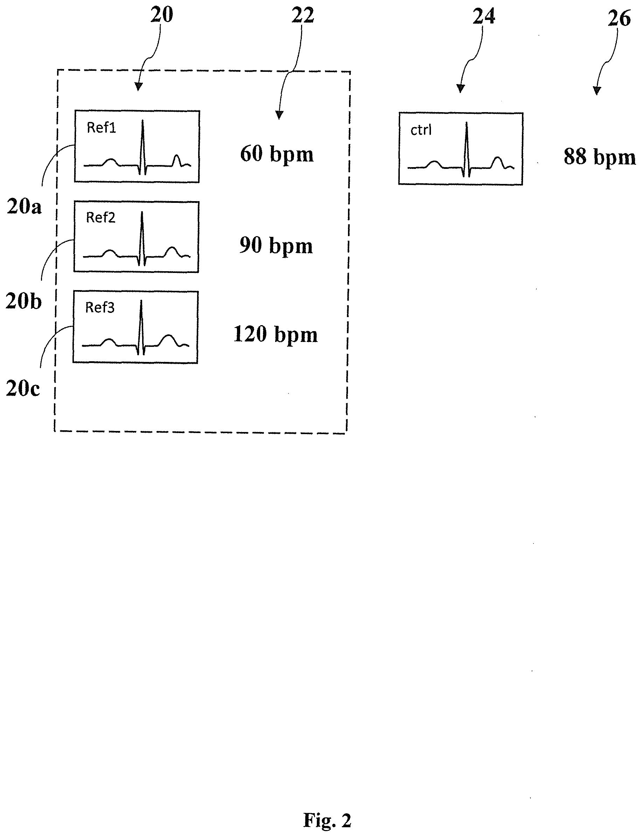

In **Fig. 2** sind schematisch Elektrokardiogramme und Werte eines Referenzparameters aus einem erfindungsgemäßen Verfahrens zur Überwachung von Biosignalen eines Probanden dargestellt.

Zum einen liegen mehrere Referenz-Elektrokardiogramme 20 des Probanden mit mehreren Herzfrequenzwerten 22 einer vor, wobei jedem der mehreren Referenz-Elektrokardiogramme 20 ein jeweiliger Herzfrequenzwert zugeordnet ist. Der jeweils zugeordnete Wert entspricht derjenigen Herzfrequenz, bei der das jeweilige Referenz-Elektrokardiogramm gemessen wurde. So ist dem ersten Referenz-Elektrokardiogramm hier beispielsweise der Herzfrequenzwert 60 Schläge pro Minute (in der Einheit bpm, beats per minute) zugeordnet. Alternativ kann ein Herzfrequenzwert in einer anderen Einheit angegeben werden, z.B. in Hertz, wobei 1 Hz einer Herzfrequenz von 60 Schlägen pro Minute entspricht. Auch ein Referenzparameter "RR-Abstand", d.h. der zeitliche Abstand der R-Zacken im Elektrokardiogramm von zwei aufeinanderfolgenden Herzzyklen (beispielsweise ausgedrückt in Millisekunden), entspricht einer Herzfrequenz. Dem zweiten Referenz-Elektrokardiogramm 20b und dem dritten Referenz-Elektrokardiogramm sind die Herzfrequenzwerte 90 bpm bzw. 120 bpm zugeordnet.

Im hier dargestellten Ausführungsbeispiel besteht der Satz 20 von mehreren Referenz-Elektrokardiogrammen aus drei Elektrokardiogrammen 20a-20c. Im Allgemeinen kann der Satz von Referenz-Elektrokardiogrammen aus zwei oder mehr Elektrokardiogrammen bestehen. Durch Erhöhung der Anzahl von Referenz-Elektrokardiogrammen kann das abgedeckte Spektrum von Referenzparameterwerten verbreitert werden, beispielsweise um nicht nur den Bereich zwischen 60 bpm und 120 bpm abzudecken, sondern auch niedrigere Herzfrequenzen, wie 50 bpm, und/oder höhere Herzfrequenzen, wie 150 bpm, einzuschließen.

Alternativ oder zusätzlich kann durch Erhöhung der Anzahl von Referenz-Elektrokardiogrammen die Auflösung innerhalb des abgedeckten Spektrums erhöht werden. So können anstatt der hier im dargestellten Ausführungsbeispiel vorliegenden Abstände zwischen Herzfrequenzwerten von 30 bpm vergleichsweise niedrigere Abstände, z.B. von 15, 10, 5 oder 1 bpm erreicht werden. Hierdurch kann eine genauere oder zuverlässigere Überwachung der Elektrokardiogramme erreicht werden.

Die mehreren Referenz-Elektrokardiogramme 20 unterscheiden sich im vorliegenden Fall insbesondere durch Position und Form der T-Welle, d.h. der Erregungsrückbildung am rechten Ende des Elektrokardiogramms. Dementsprechend weisen die mehreren Referenz-Elektrokardiogramme unterschiedliche QT-Zeiten auf, wobei die QT-Zeit bei dem ersten Referenz-Elektrokardiogramm 20a größer ist als bei den anderen beiden Referenz-Elektrokardiogrammen 20b, 20c und wobei die QT-Zeit bei dem dritten Referenz-Elektrokardiogramm 20c niedriger ist als bei den Referenz-Elektrokardiogrammen 20a, 20b. Dies ist im Einklang mit einer physiologischen Verringerung der QT-Zeit mit zunehmender Herzfrequenz. Die mehreren Referenz-Elektrokardiogramme 20 wurden bei einem gesunden Probanden gemessen.

Ferner liegt ein Kontroll-Elektrokardiogramm 24 des Probanden mit einem ihm zugeordneten Herzfrequenzwert 26 von 88 bpm vor. Das Kontroll-Elektrokardiogramm 24 wurde an dem gleichen Probanden zu einem späteren Zeitpunkt zur Überwachung der Herzfunktion gemessen. In Verbindung mit der Messung des Kontroll-Elektrokardiogramms 24 wurde zudem der Herzfrequenzwert 26 bestimmt.

In einem erfindungsgemäßen Verfahren kann nun das Kontroll-Elektrokardiogramm 24 mit mindestens einem der mehreren Referenz-Elektrokardiogramme 20 verglichen werden.

Hierzu wird ein Vergleichs-Elektrokardiogramm aus den mehreren Referenz-Elektrokardiogrammen 20 ausgewählt. Diese Auswahl basiert auf den mehreren Herzfrequenzwerten 22, die den Referenz- Elektrokardiogrammen 20 zugeordnet sind, sowie auf dem Herzfrequenzwert 26, der dem Kontroll-Elektrokardiogramm 24 zugeordnet ist. Bevorzugt kann dasjenige der mehreren Referenz-Elektrokardiogramme 20 als Vergleichs-Elektrokardiogramm ausgewählt werden, dessen zugeordneter Herzfrequenzwert 22 zu dem Herzfrequenzwert 26 (der dem Kontroll-Elektrokardiogramm 24 zugeordnet ist) die geringste Differenz aufweist (wobei die Differenz als Absolutbetrag ausgedrückt wird). Hier weist das zweite Referenz-Elektrokardiogramm 20b eine Differenz von 2 bpm auf, wohingegen die Referenz-Elektrokardiogramme 20a und 20c Differenzen in Höhe von 28 bpm bzw. 32 bpm zur Herzfrequenz 26 des Kontroll-Elektrokardiogramms 24 aufweisen. Folglich weist das zweite Referenz-Elektrokardiogramm 20b die geringste Differenz der Herzfrequenz auf. Das zweite Referenz-Elektrokardiogramm 20b wird als Vergleichs-Elektrokardiogramm zum Vergleich mit dem Kontroll-Elektrokardiogramm 24 ausgewählt. In anderen Ausführungsformen kann dasjenige der mehreren Referenz-Elektrokardiogramme 20 als Vergleichs-Elektrokardiogramm ausgewählt werden, dessen zugeordneter Herzfrequenzwert 22 den nächstniedrigeren bzw. nächsthöheren Wert zu dem Herzfrequenzwert 26 (der dem Kontroll-Elektrokardiogramm 24 zugeordnet ist) darstellt. Dies ist im dargestellten Fall das erste Referenz-Elektrokardiogramm 20a bzw. das zweite Referenz-Elektrokardiogramm 20b.

Im vorliegenden Fall weist das Kontroll-Elektrokardiogramm keine veränderte QT-Zeit oder eine andere wesentliche Veränderung gegenüber dem Vergleichs-Elektrokardiogramm 20b auf. Es kann daher als unauffällig gegenüber dem Vergleichs-Elektrokardiogramm eingestuft werden.

Bei einem nicht erfindungsgemäßen Verfahren zur Überwachung von Elektrokardiogrammen, wobei beispielsweise nicht mehrere Referenz-Elektrokardiogramme vorliegen, würde ein Kontroll-Elektrokardiogramm mit dem einzigen vorliegenden Referenz-Elektrokardiogramm, welches beispielsweise wie Elektrokardiogramm 20a bei einer Herzfrequenz von 60 bpm gemessen wurde, verglichen werden. Hierbei würde eine Abweichung der QT-Zeit festgestellt werden, ohne jedoch Rückschlüsse auf die Ursache (z.B. physiologische Frequenzabhängigkeit oder pathologische Ursache) zu ermöglichen. Falls dennoch eine Diagnose aufgrund der Abweichung gestellt würde, so erhöht sich insbesondere das Risiko von falsch-positiven Ergebnissen. Falls dem dort einzigen vorliegenden Referenz-Elektrokardiogramm eine Information über die zugrundeliegende Herzfrequenz (z.B. 60 bpm) beigefügt ist, so würde zwar zumindest eine frequenzabhängige Anpassung des QT-Zeitparameters ermöglicht werden, wobei diese beispielsweise auf einem mathematischen oder statistischem Modell beruhen kann, welches die genauen Gegebenheiten des Individuums nur unvollständig berücksichtigen kann. Beispiele von frequenzabhängigen Anpassungen der QT-Zeit sind die Hegglin-Formel, Bazett-Formel oder die Fridericia-Formel. Die Veränderungen im Elektrokardiogramm können von Proband zu Proband variieren und können daher nicht verallgemeinert werden.

Im Vergleich hierzu wird in manchen erfindungsgemäßen Ausführungsbeispielen, insbesondere bei geringer Differenz der Herzfrequenzwerte wie in dem in Fig. 2 dargestellten Fall, eine frequenzabhängige Anpassung des QT-Zeitparameters überflüssig.

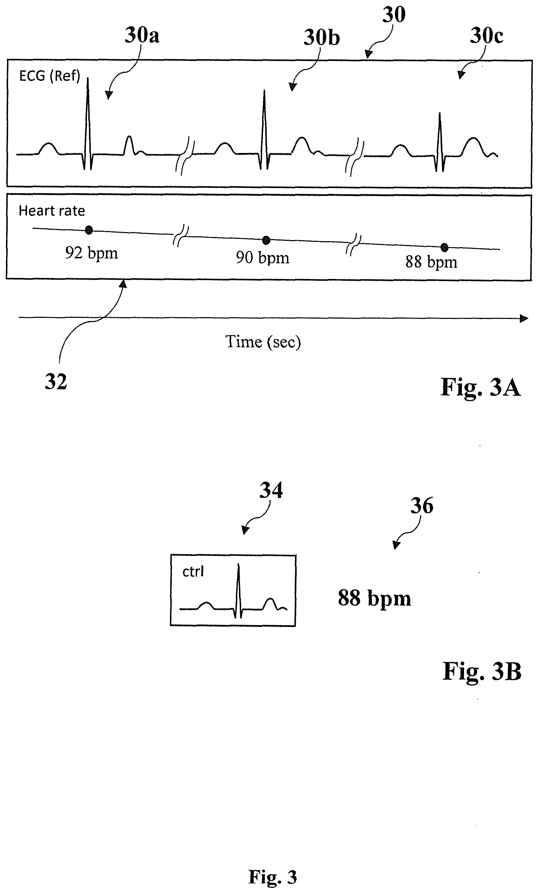

In **Fig. 3** sind schematisch Elektrokardiogramme und Werte eines Referenzparameters dargestellt, wie sie im Laufe eines weiteren Ausführungsbeispiels eines erfindungsgemäßen Verfahrens zur Überwachung von Biosignalen eines Probanden vorliegen können. **Fig. 3A** zeigt mehrere Referenz-Elektrokardiogramme 30 und mehrere Herzfrequenzwerte 32 als Referenzparameter.

Die mehreren Referenz-Elektrokardiogramme 30 des Probanden sind als kontinuierliche Zeitreihe gemessen. Die mehreren Herzfrequenzwerte 32 sind simultan zu oder in synchronisierter Art mit den mehreren Referenz-Elektrokardiogrammen ebenfalls als kontinuierliche Zeitreihe gemessen. Der dargestellte Verlauf der Herzfrequenzen beruht auf einer Interpolation, welche eine quasi-kontinuierliche Messreihe mittels Datenverarbeitung in eine im strikten Sinne kontinuierliche Zeitreihe überführt.

Die mehreren Herzfrequenzwerte 32 sind den mehreren Referenz-Elektrokardiogrammen 30 mittels der synchronisierten Aufnahme und der somit gemeinsamen Zeitachse zugeordnet.

Die mehreren Referenz-Elektrokardiogrammen 30 und die mehreren Herzfrequenzwerte 32 des in Fig. 3A dargestellten Ausführungsbeispiels können beispielsweise in Folge von Reizen, die die Herzfrequenz erhöhen, wie einer gezielten Belastung des Probanden auf einem Ergometer, gemessen worden sein. Der Proband hat (beispielsweise durch ein erfindungsgemäßes System) die Anweisung erhalten, sich auf dem Ergometer sich bis zu seiner Maximalbelastung oder über eine definierte Zeit zu verausgaben. Unmittelbar nach Abschluss der Belastung befindet sich seine Herzfrequenz an einem Maximum. Andere Formen der Belastung zur anfänglichen Erhöhung der Herzfrequenz sind Treppensteigen oder Kniebeugen mit einer bestimmten Anzahl von Wiederholungen. Zusätzlich oder alternativ kann die Herzfrequenz medikamentös erhöht werden.

Nach Erreichen einer maximalen Herzfrequenz und Beendigung der körperlichen Belastung wurde die Messung von Referenz-Elektrokardiogrammen gestartet. Gleichzeitig wurde die Herzfrequenz aufgezeichnet. Die Herzfrequenz kann direkt aus den aufgezeichneten Referenz-Elektrokardiogrammen, nämlich mittels des RR-Abstands, erfolgen. Es ist somit keine zusätzliche Sensoranordnung (z.B. Pulsometer) notwendig. Ferner wird die Synchronisierung der beiden Zeitreihen durch die direkte Ermittlung aus der Zeitreihe der gemessenen Referenz-Elektrokardiogramme erleichtert.

Im Laufe der Zeit nimmt die Herzfrequenz ausgehend von dem Maximum bis zum Ruhepuls langsam ab. In diesem Ausführungsbeispiel kann ein breites Spektrum von Herzfrequenzen vom Maximalpuls bis zum Ruhepuls aufgenommen werden. Je nach Geschwindigkeit der Abnahme der Herzfrequenz ist es gegebenenfalls möglich, zu jeder ganzzahligen Herzfrequenz im abgedeckten Bereich ein Referenz-Elektrokardiogramm aufzunehmen und diesem zuzuordnen. Falls dies der Fall ist, so kann für die spätere Auswahl zum Vergleich mit einem Kontroll-Elektrokardiogramm Identität der Herzfrequenzwerte gefordert werden. Zu Illustrationszwecken ist in Fig. 3A lediglich ein Ausschnitt der Zeitreihen dargestellt, nämlich drei Bereiche bei etwa 90 Schlägen pro Minute. Dem Referenz-Elektrokardiogramm 30a ist hierbei die Herzfrequenz 92bpm zugeordnet. Dem Referenz-Elektrokardiogramm 30b ist die Herzfrequenz 90bpm zugeordnet. Dem Referenz-Elektrokardiogramm 30c ist die Herzfrequenz 88bpm zugeordnet.

Alternativ oder zusätzlich kann in manchen Ausführungsbeispielen die Messung von Referenz-Elektrokardiogrammen während des Herzfrequenzanstiegs während einer Belastung, z.B. auf einem Ergometer, erfolgen.

In **Fig. 3B** ist ein Kontroll-Elektrokardiogramm 34 dargestellt, welches an dem gleichen Probanden zu einem späteren Zeitpunkt zur Überwachung gemessen wurde. Gleichzeitig wird ein Herzfrequenzwert 36 von 88 Schlägen pro Minute bestimmt und dem Kontroll-Elektrokardiogramm 34 zugeordnet.

Durch Vergleich wird derjenige Herzfrequenzwert aus den mehreren Herzfrequenzen 32 bestimmt, der mit dem Herzfrequenzwert 36 identisch ist oder die geringste Differenz aufweist. Im Fall des dargestellten Ausführungsbeispiels sind dem Referenz-Elektrokardiogramm 30c und dem Kontroll-Elektrokardiogramm 34 identische Herzfrequenzwerte, nämlich 88 Schläge pro Minute, zugeordnet. Das Referenz-Elektrokardiogramm 30c wird als Vergleichs-Elektrokardiogramm ausgewählt und mit dem Kontroll-Elektrokardiogramm 34 verglichen.

Dies erlaubt eine effektive Überwachung eines Probanden, insbesondere eines Sportlers während des Trainings oder eines Rekonvaleszenten während der Rehabilitation: Im Rahmen eines Belastungs-EKG ändert sich die Herzfrequenz. Gemäß der Lehre der vorliegenden Erfindung, insbesondere in dem hier dargestellten Ausführungsbeispiel, ist es möglich zu jedem Kontroll-Elektrokardiogramm im Laufe des Belastungs-EKG ein passendes Referenz-Elektrokardiogramm auszuwählen, also ein unter den gleichen oder ähnlichen Bedingungen gemessenes Referenz-Elektrokardiogramm.

Damit können Belastungs-induzierte Veränderungen der EKG-Kurve von Veränderungen mit anderen Ursachen (z.B. pathologischen Ursachen wie einer Durchblutungsstörung) unterschieden werden.

Falls zwei der mehreren Herzfrequenzwerte 32 die gleiche Differenz zu dem Herzfrequenzwert 36 aufweisen, so können beide zugeordneten Elektrokardiogramme als Vergleichs-Elektrokardiogramme ausgewählt werden und zum Vergleich herangezogen werden. Alternativ kann ein mittleres Vergleichs-Elektrokardiogramm aus den beiden ausgewählten Referenz-Elektrokardiogrammen gebildet werden. Ferner alternativ kann eines der beiden Referenz-Elektrokardiogramme zufällig oder in vorbestimmter Art (beispielsweise: das mit dem geringeren/höheren zugeordneten Wert des Referenzparameters; oder das zeitlich jüngere bei unterschiedlichen Aufnahmedaten) als Vergleichs-Elektrokardiogramm ausgewählt werden.

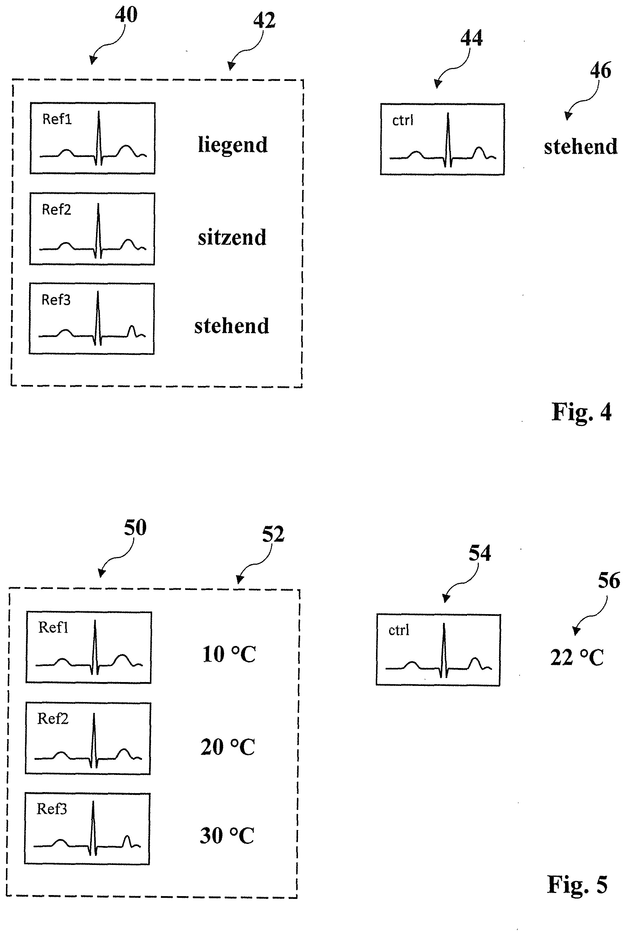

In **Fig. 4** sind drei Referenz-Elektrokardiogramme 40 eines Probanden mit zugeordneten Werten 42 des Referenzparameters "Körperhaltung", sowie ein Kontroll-Elektrokardiogramm 44 des gleichen Probanden mit zugeordnetem Wert 46 des Referenzparameters "Körperhaltung" schematisch dargestellt.

Der Referenzparameter "Körperhaltung" ist ein Referenzparameter, der nicht-numerische Werte annimmt. In einer computer-basierten Implementierung können jedem dieser nichtnumerischen Werte einer Codierung entsprechende numerische oder binäre Werte zugeordnet werden. Zur Veranschaulichung werden hier die Werte in ihrer beschreibenden Wortform dargestellt. Der Referenzparameter "Körperhaltung" nimmt hier beispielsweise die Werte liegend, sitzend oder stehend an.

Der Referenzparameter "Körperhaltung" hat mittels des sog. Lagetyps Einfluss auf die Form von Elektrokardiogrammen, wie im Folgenden erläutert. Elektrokardiogramme können mittels der verschiedenen oben genannten Ableitungen als Projektionen eines Dipols im Körper des Probanden aufgefasst werden. Bei gegebenen Elektrodenpositionen, z.B. den in Fig. 1A dargestellten, hängt ein gemessenes Elektrokardiogramm unter anderem von der Orientierung des zu projizierenden Dipols ab. Die Orientierungen werden gemeinhin unter dem Begriff des Lagetyps, beispielsweise in Verbindung mit dem Cabrerakreis, beschrieben. Beispiele von Lagetypen umfassen den Normtyp, Linkstyp und Rechtstyp. Am Beispiel der Ableitung nach Einthoven I (also zwischen linkem und rechtem Arm) ist festzustellen, dass diese bei einem Linkstyp eine starke positive R-Zacke zeigt, wohingegen die R-Zacke (in der Ableitung nach Einthoven I) bei einem Rechtstyp sogar negativ sein kann. Entsprechende Lagetyp-abhängige Veränderungen finden sich auch in den weiteren Ableitungen und der anderen Charakteristiken des Elektrokardiogramms (Form der P-Welle, T-Welle, etc.). Veränderungen des Lagetyps können pathologische Ursachen haben, z.B. eine Hypertrophie. So führt beispielsweise eine linksventrikuläre Hypertrophie (LVH) zu einer Verschiebung vom Normtyp zum Linkstyp. Gleichzeitig hat jedoch auch die Körperhaltung Einfluss auf den Lagetyp. So kann Aufstehen zu Veränderungen des Elektrokardiogramms führen, die einem Rechtstyp ähneln. Der Vergleich von Elektrokardiogrammen, die in unterschiedlicher Körperhaltung gemessen wurden, kann somit zu falschen oder unbegründeten Vergleichsergebnissen führen. Insbesondere bei Langzeit-EKG-Untersuchungen kann es zudem vorkommen, dass der Proband seine Körperhaltung im Laufe der Messung verändert.

In dem Ausführungsbeispiel von Fig. 4 wird daher für den Vergleich mit dem Kontroll-Elektrokardiogramm eines der Referenz-Elektrokardiogramme 40 ausgewählt, dessen zugeordneter Körperhaltungs-Wert 42 mit dem des Kontroll-Elektrokardiogramms 44 (also "stehend") übereinstimmt. Somit wird vermieden, dass Elektrokardiogramme, die in unterschiedlicher Körperhaltung gemessen wurden (und deswegen einen anderen Lagetyp aufweisen), miteinander verglichen werden.

**Fig. 5** zeigt drei gemessene Referenz-Elektrokardiogramme 50 eines Probanden mit zugeordneten Werten 52 des Referenzparameters "Umgebungstemperatur", sowie ein gemessenes Kontroll-Elektrokardiogramm 54 des Probanden mit zugeordnetem Wert 56 des Referenzparameters "Umgebungstemperatur".

Werte des Referenzparameters "Umgebungstemperatur" beschreiben zumindest teilweise Randbedingungen der Umgebung oder Umwelt des Probanden während der Messung.

Basierend auf dem Umgebungstemperaturwert 56, der dem Kontroll-Elektrokardiogramm 54 zugeordnet ist, und auf den mehreren Umgebungstemperaturwerten 52, die den mehreren Referenz-Elektrokardiogrammen 50 zugeordnet sind, kann ein Vergleichs-Elektrokardiogramm ausgewählt werden. Im vorliegenden Fall eines Umgebungstemperaturwerts 56 von 22°C ist der nächstliegende unter den Werten 52 der Referenz-Elektrokardiogramme 50 die Umgebungstemperatur von 20°C. Diese ist dem zweiten der drei Referenz-Elektrokardiogramme 50 zugeordnet. Dieses zweite der Referenz-Elektrokardiogramme 50 wird somit als Vergleichs-Elektrokardiogramm ausgewählt und mit dem Kontroll-Elektrokardiogramm 56 verglichen. Somit wird vermieden, dass Elektrokardiogramme, die unter unterschiedlichen Randbedingungen der Umgebung gemessen wurden, miteinander verglichen werden.

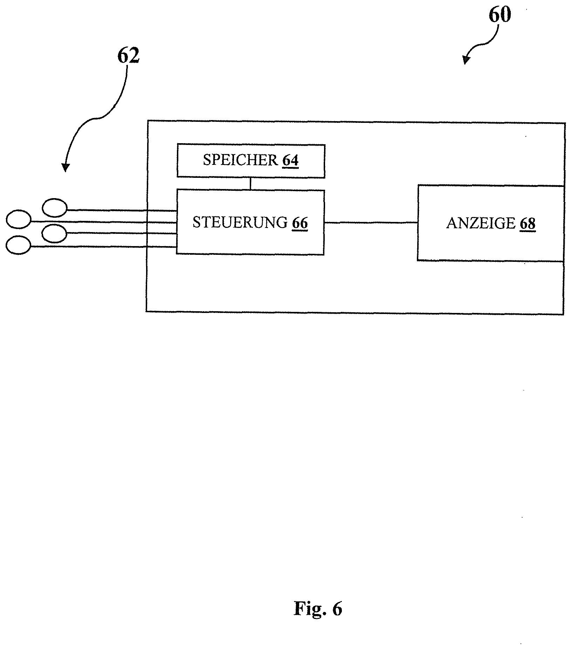

**Fig. 6** zeigt ein Ausführungsbeispiel eines Systems 60 zur Überwachung von Elektrokardiogrammen eines Probanden mit vier Sensoren 62, einem Speicher 64, einer Steuerung 66 und einer Anzeige 68.

Die Sensoren 62 sind als EKG-Elektroden ausgebildet und dazu ausgelegt, ein Kontroll-Elektrokardiogramm zu messen. Hierzu können sie insbesondere gemäß Fig. 1 an dem Probanden angeordnet werden. Ferner sind die Sensoren 62 dazu ausgelegt, mehrere Referenz-Elektrokardiogramme zu messen.

Der Speicher 64 ist dazu ausgelegt, die mehreren Referenz-Elektrokardiogramme zu speichern. Jedem der mehreren Referenz-Biosignalen ist in dem Speicher ein jeweiliger Wert eines Referenzparameters zugeordnet. Die Speicherung und Zuordnung von Referenz-Elektrokardiogrammen und Referenzparameterwerten kann beispielsweise in einem Datenbankformat erfolgen.

Die Steuerung 66 ist dazu eingerichtet, einen dem Kontroll-Elektrokardiogramm zuzuordnenden Wert des Referenzparameters zu bestimmen. Hier umfasst die Bestimmung des Werts des Referenzparameters eine Messung mittels der Sensoren 62.

Ferner ist die Steuerung 66 dazu eingerichtet, ein Vergleichs-Elektrokardiogramm, aus den mehreren gespeicherten Referenz-Elektrokardiogrammen, basierend auf dem bestimmten Wert des Referenzparameters, der dem Kontroll-Elektrokardiogramm zugeordnet ist, und auf den mehreren Werten des Referenzparameters, die den mehreren Referenz-Elektrokardiogrammen zugeordnet sind, auszuwählen.

Ferner ist die Steuerung 66 dazu eingerichtet, das Kontroll-Elektrokardiogramm und das Vergleichs-Elektrokardiogramm zu vergleichen.

Die Anzeige 68 ist dazu ausgelegt, Informationen auszugeben. Die Anzeige 68 ist als LCD-Bildschirm ausgestaltet, um die Information optisch auszugeben. Insbesondere ist sie dazu ausgelegt, auszugeben, ob ein Verfahren zur Überwachung von Elektrokardiogrammen ordnungsgemäß durchgeführt werden konnte. Ferner ist sie dazu ausgelegt, nach ordnungsgemäßer Durchführung des Verfahrens eine Handlungsempfehlung basierend auf dem Vergleich von Kontroll-Elektrokardiogramm und Vergleichs-Elektrokardiogramm auszugeben. Eine auszugebende Handlungsempfehlung kann beispielsweise auf das Hinsetzen, das Ausruhen oder die Aufnahme einer sportlichen Betätigung des Probanden gerichtet sein.

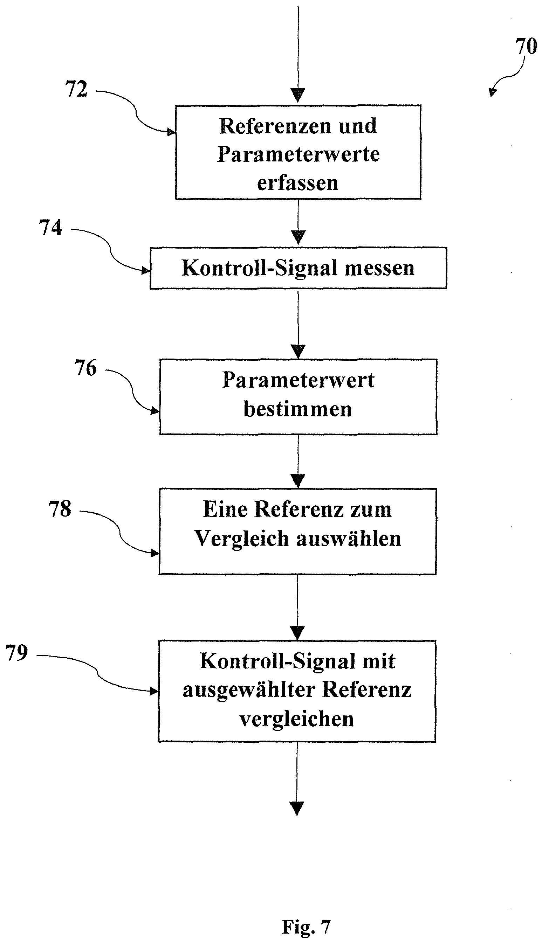

**Fig. 7** zeigt ein Ablaufdiagramm eines Verfahrens 70 gemäß einem Ausführungsbeispiels. Das Verfahren 70 dient der zur Überwachung von Biosignalen eines Probanden.

Es umfasst die Schritte des Erfassens 72 von mehreren Referenz-Biosignalen des Probanden, jeweils mit einem Wert eines Referenzparameters, des Messens 74 eines Kontroll-Biosignals des Probanden, des Bestimmens 76 eines Werts des Referenzparameters, des Auswählens 78 eines Vergleichs-Biosignals aus den mehreren gespeicherten Referenz-Biosignalen und des Vergleichens 79 des gemessenen Kontroll-Biosignals mit dem ausgewählten Vergleichs-Biosignal.

Das Auswählen 78 eines Vergleichs-Biosignals aus den mehreren erfassten Referenz-Biosignalen basiert auf dem in Schritt 76 bestimmten Wert des Referenzparameters und auf den mehreren Werten des Referenzparameters, die in Schritt 72 erfasst wurden.

Werte des Referenzparameters beschreiben physiologische Zustände des Probanden und/oder Randbedingungen einer Umgebung zumindest teilweise und setzen keine Diagnose eines pathologischen, insbesondere eines kardialen, Ereignisses voraus.

## Patentansprüche

1. Verfahren (70) zur Überwachung von Biosignalen eines Probanden, umfassend die Schritte:
- Erfassen (72) von mehreren Referenz-Biosignalen, insbesondere Referenz-Elektrokardiogrammen (30), des Probanden, wobei jedem der mehreren Referenz-Biosignalen ein jeweiliger Wert eines Referenzparameters zugeordnet wird, wobei der Referenzparameter eine Herzfrequenz (32) umfasst,
- Messen (74) eines Kontroll-Biosignals, insbesondere eines Kontroll-Elektrokardiogramms (34), des Probanden, mittels eines Sensors,
- Bestimmen (76) eines Werts (36) des Referenzparameters, der dem Kontroll-Biosignal zugeordnet wird,
- Auswählen (78) mindestens eines Vergleichs-Biosignals, insbesondere mindestens eines Vergleichs-Elektrokardiogramms (30c), aus den mehreren gespeicherten Referenz-Biosignalen (30), basierend auf dem Wert des Referenzparameters, der dem Kontroll-Biosignal zugeordnet ist, und auf den mehreren Werten des Referenzparameters, die den mehreren Referenz-Biosignalen zugeordnet sind,
- Vergleichen (79) des gemessenen Kontroll-Biosignals mit dem ausgewählten Vergleichs-Biosignal, und
- Ausgeben einer Handlungsempfehlung basierend auf einer Abweichung zwischen Kontroll-Biosignal und Vergleichs-Biosignal,
wobei Werte des Referenzparameters physiologische Zustände des Probanden zumindest teilweise beschreiben,
**dadurch gekennzeichnet, dass** die mehreren Referenz-Biosignale kontinuierlich als mehrere Intervalle einer Zeitreihe vorliegen.

2. Verfahren gemäß Anspruch 1, wobei dasjenige der Referenz-Biosignale als Vergleichs-Biosignal ausgewählt wird, dessen zugeordneter Wert des Referenzparameters mit dem dem Kontroll-Biosignal zugeordneten Wert des Referenzparameters identisch ist oder die geringste Differenz aufweist oder dessen zugeordneter Wert des Referenzparameters den nächsthöheren oder den nächstniedrigeren Wert zu dem dem Kontroll-Biosignal zugeordneten Wert des Referenzparameters darstellt.

3. Verfahren gemäß einem der vorstehenden Ansprüche, wobei der Referenzparameter mehrere Komponenten umfasst.

4. Verfahren gemäß einem der vorstehenden Ansprüche, wobei das gemessene Kontroll-Biosignal mit dem ihm zugeordnetem Wert des Referenzparameters als weiteres der mehreren Referenz-Biosignale mit zugeordnetem Wert des Referenzparameters aufgenommen wird.

5. Verfahren gemäß einem der vorstehenden Ansprüche, wobei die Biosignale Elektrokardiogramme sind oder wobei die Biosignale EKG-Parameter sind, die aus einem Elektrokardiogramm ermittelt werden können, so wie QT-Zeitintervall, Zeiten und/oder Amplituden im QRS-Komplex, ST-Strecke, P-Welle, R-Zacke, T-Welle, Q-Zacke, S-Zacke, U-Welle, Nulllinie.

6. Verfahren gemäß einem der vorstehenden Ansprüche, wobei der Referenzparameter ferner einen oder mehrere der folgenden umfasst:
- aus einem Elektrokardiogramm ermittelbare EKG-Parameter, Blutzuckerspiegel, Blutdruck, Sauerstoffgehalt im Blut, Elektrolyt-Spiegel, Körpertemperatur, Atemfrequenz, Atemminutenvolumen;
- Medikation des Probanden, Körperhaltung des Probanden, aktueller und/oder bisheriger Bewegungszustand des Probanden, Lebensgewohnheiten des Probanden, insbesondere dessen Berufstätigkeit, Essgewohnheiten, Häufigkeit sportlicher Betätigung.

7. Verfahren gemäß einem der vorstehenden Ansprüche, wobei Werte des Referenzparameters ferner Randbedingungen einer Umgebung zumindest teilweise beschreiben.

8. Verfahren gemäß dem vorstehenden Anspruch, wobei der Referenzparameter ferner einen oder mehrere der folgenden umfasst: Tageszeit, Umgebungstemperatur, Luftdruck, Luftfeuchtigkeit, Jahreszeit.

9. Verfahren gemäß einem der vorstehenden Ansprüche, wobei das Ausgeben ferner mindestens eine der folgenden Informationen ausgibt:
der Wert des Referenzparameters, der dem Kontroll-Biosignal zugeordnet ist; der Wert des Referenzparameters, der dem ausgewählten Vergleichs-Biosignal zugeordnet ist; das Kontroll-Biosignal; das Vergleichs-Biosignal; eine Abweichung zwischen Kontroll-Biosignal und Vergleichs-Biosignal; eine Abweichung zwischen dem Wert des Referenzparameters, der dem Kontroll-Biosignal zugeordnet ist, und dem Wert des Referenzparameters, der dem ausgewählten Vergleichs-Biosignal zugeordnet ist.

10. Verfahren gemäß einem der vorstehenden Ansprüche, wobei die Biosignale eines der folgenden sind:
Blutdruckkurven, Blutbilder, Elektrokardiogramme, Elektroenzephalogramme, Elektromyogramme, Elektroretinogramme, Aufzeichnungen der Atemgaszusammensetzung, Aufzeichnungen der Zusammensetzung von Ausscheidungen.

11. Computerprogramm-Produkt, das auf einem computerlesbaren Medium gespeichert ist und mit Programmcode, der eine Vorrichtung gemäss Anspruch 12 veranlasst, die Schritte des in einem der Ansprüche 1 bis 10 definierten Verfahrens durchzuführen.

12. System (60) zur Überwachung von Biosignalen eines Probanden, umfassend:
- Mindestens einen Sensor (62), insbesondere EKG-Elektroden, zum Messen eines Kontroll-Biosignals, insbesondere eines Kontroll-Elektrokardiogramms, des Probanden;
- Speicher (64) zum Speichern von mehreren Referenz-Biosignalen, insbesondere Referenz-Elektrokardiogrammen, des Probanden, wobei jedem der mehreren Referenz-Biosignale ein jeweiliger Wert eines Referenzparameters zugeordnet ist, wobei Werte des Referenzparameters physiologische Zustände des Probanden zumindest teilweise beschreiben und wobei die mehreren Referenz-Biosignale kontinuierlich als mehrere Intervalle einer Zeitreihe vorliegen;
- Steuerung (66), dazu eingerichtet,
∘ Einen dem Kontroll-Biosignal zugeordneten Wert des Referenzparameters zu bestimmen, wobei der Referenzparameter eine Herzfrequenz umfasst,
∘ mindestens ein Vergleichs-Biosignal, insbesondere mindestens ein Vergleichs-Elektrokardiogramm, aus den mehreren gespeicherten Referenz-Biosignalen, basierend auf dem bestimmten Wert des Referenzparameters und den mehreren Werten des Referenzparameters, die den mehreren Referenz-Biosignalen zugeordnet sind, auszuwählen,
∘ das Kontroll-Biosignal und das Vergleichs-Biosignal zu vergleichen, und
∘ eine Handlungsempfehlung basierend auf einer Abweichung zwischen Kontroll-Biosignal und Vergleichs-Biosignal auszugeben.

## Claims

1. A method (70) for monitoring biosignals of a test subject, comprising,
- recording (72) a plurality of reference biosignals, in particular reference electrocardiograms (30), of the test subject, wherein each of the plurality of reference biosignals is assigned with a respective value of a reference parameter, wherein the reference parameter comprises a heart rate;
- measuring (74) a control biosignal, in particular a control electrocardiogram (34), of the test subject, using a sensor,
- determining (76) a value (36) of the reference parameter assigned with the control biosignal,
- selecting (78) at least one comparative biosignal, in particular at least one comparative electrocardiogram (30c), from the plurality of stored reference biosignals (30), based on a value of the reference parameter assigned to the control biosignal, and on a plurality of values of the reference parameter assigned to the plurality of reference biosignals,
- comparing (76) the measured control biosignal with the selected comparative biosignal, and
- outputting an action recommendation based on a deviation between the control biosignal and the comparative biosignal,
wherein the values of the reference parameter describe at least partially a physiological state of the test subject,
**characterized in that** the plurality of reference biosignals are provided continuously as multiple intervals of a time series.

2. The method according to claim 1, wherein among the plurality of reference biosignals, a reference biosignal having an assigned value of the reference parameter representing a value identical to or having the smallest difference with respect to the value of the reference parameter assigned to the control biosignal, or having an assigned value of the reference parameter representing the next higher or the next lower value with respect to the value of the reference parameter assigned to the control biosignal is selected as the comparative biosignal.

3. The method according to one of the preceding claims, wherein the reference parameter comprises a plurality of components.

4. The method according to one of the preceding claims, wherein the measured control biosignal having an assigned value of the reference parameter is recorded as another plurality of reference biosignals having an assigned value of the reference parameter.

5. The method according to one of the preceding claims, wherein the biosignal is an electrocardiogram, or the biosignal is an ECG-parameter that can be determined from an electrocardiogram, such as, a QT-time interval, times and/or amplitudes in a QRS-complex, ST-segment, P-wave, R-spike, T-wave, Q-spike, S-spike, U-wave, and zero-line.

6. The method according to one of the preceding claims, wherein the reference parameter further comprises one or more of the following:
- ECG-parameters that can be determined from an electrocardiogram, blood sugar level, blood pressure, oxygen content in blood, electrolyte levels, body temperature, respiratory rate, minute ventilation;
- medication of the test subject, posture of the test subject, current and/or motion state of the test subject up till now, habits of the test subject, in particular occupation, eating habits, frequency of sports activity thereof;

7. The method according to one of the preceding claims, wherein values of the reference parameter furthermore at least partially describe boundary conditions of an environment.

8. A method according to the preceding claim, wherein the reference parameter further comprises one or more of the following: time of day, ambient temperature, air pressure, humidity, season.

9. The method according to one of the preceding claims , wherein the outputting further outputs at least one of the following information: the value of the reference parameter assigned to the control biosignal; the value of the reference parameter assigned to the selected comparative biosignal; the control biosignal; the comparative biosignal; a deviation between the control biosignal and the comparative biosignal; a deviation between the value of the reference parameter assigned to the control biosignal and the value of the reference parameter assigned to the selected comparative biosignal.

10. The method according to one of the preceding claims, wherein the biosignal is one of, a blood pressure curve, hemogram, electrocardiogram, electroencephalogram, electromyogram, electroretinogram, record of respiratory gas composition, record of excretion composition.

11. A computer program product stored on a computer readable medium and using a program code causes an apparatus according to claim 12 to perform the steps of the method defined by any one of claims 1 to 10.

12. A system (60) for monitoring biosignals of a test subject, comprising:
- at least one sensor (62), in particular ECG electrodes, for measuring a control biosignal, in particular a control electrocardiogram of the test subject;
- a memory (64) for storing a plurality of reference biosignals, in particular reference electrocardiograms, of the test subject, wherein each of the plurality of reference biosignals is assigned with a respective value of a reference parameter, wherein values of the reference parameter describe at least partially a physiological state of the test subject, and wherein the plurality of reference biosignals are provided continuously as multiple intervals of a time series;
- a controller, wherein the controller is configured to,
- determine a value of the reference parameter assigned to the control biosignal, wherein the reference parameter comprises a heart rate,
- select at least one comparative biosignal, in particular at least one comparative electrocardiogram, from the plurality of stored reference biosignals, based on the determined value of the reference parameter and on the plurality of values of the reference parameter assigned to the plurality of reference biosignals,
- compare the control biosignal and the comparative biosignal, and
- output an action recommendation based on a deviation between the control biosignal and the comparative biosignal.

## Revendications

1. Procédé (70) pour la surveillance de signaux biologiques d'un sujet d'expérience, comprenant les étapes :
- d'acquisition (72) de plusieurs signaux biologiques de référence, en particulier électrocardiogrammes de référence (30), du sujet d'expérience, dans lequel une valeur respective d'un paramètre de référence est associée à chacun des plusieurs signaux biologiques de référence, dans lequel le paramètre de référence comprend une fréquence cardiaque (32),
- de mesure (74) d'un signal biologique de contrôle, en particulier d'un électrocardiogramme de contrôle (34), du sujet d'expérience, au moyen d'un capteur,
- de détermination (76) d'une valeur (36) du paramètre de référence qui est associée au signal biologique de contrôle,
- de sélection (78) au moins d'un signal biologique de comparaison, en particulier au moins d'un électrocardiogramme de comparaison (30c), à partir des plusieurs signaux biologiques de référence (30) mis en mémoire, sur la base de la valeur du paramètre de référence qui est associée au signal biologique de contrôle, et des plusieurs valeurs du paramètre de référence qui sont associées aux plusieurs signaux biologiques de référence,
- de comparaison (79) du signal biologique de contrôle mesuré au signal biologique de comparaison sélectionné, et
- de délivrance d'une recommandation de manipulation sur la base d'un écart entre le signal biologique de contrôle et le signal biologique de comparaison,
dans lequel des valeurs du paramètre de référence décrivent au moins en partie des états physiologiques du sujet d'expérience,
**caractérisé en ce que** les plusieurs signaux biologiques de référence se présentent en continu comme plusieurs intervalles d'une série chronologique.

2. Procédé selon la revendication 1, dans lequel celui des signaux biologiques de référence dont la valeur du paramètre de référence associée est identique à la valeur du paramètre de référence associée au signal biologique de contrôle ou présente la plus petite différence ou dont la valeur du paramètre de référence associée représente la valeur immédiatement supérieure ou la valeur immédiatement inférieure par rapport à la valeur du paramètre de référence associée au signal biologique de contrôle est sélectionné comme signal biologique de comparaison.

3. Procédé selon l'une quelconque des revendications précédentes, dans lequel le paramètre de référence comprend plusieurs composantes.

4. Procédé selon l'une quelconque des revendications précédentes, dans lequel le signal biologique de contrôle mesuré avec la valeur du paramètre de référence associée à celui-ci est capté comme autre des plusieurs signaux biologiques de référence avec la valeur du paramètre de référence associée.

5. Procédé selon l'une quelconque des revendications précédentes, dans lequel les signaux biologiques sont des électrocardiogrammes ou dans lequel les signaux biologiques sont des paramètres ECG, qui peuvent être établis à partir d'un électrocardiogramme, tels que l'intervalle QT, les durées et/ou amplitudes dans le complexe QRS, le segment ST, l'onde P, la pointe R, l'onde T, la pointe Q, la pointe S, l'onde U, la ligne plate.

6. Procédé selon l'une quelconque des revendications précédentes, dans lequel le paramètre de référence comprend en outre un ou plusieurs de ce qui suit :
- paramètres ECG pouvant être établis à partir d'un électrocardiogramme, glycémie, pression artérielle, taux de saturation en oxygène, taux d'électrolytes, température corporelle, fréquence respiratoire, volume respiratoire par minute ;
- médication du sujet d'expérience, posture du sujet d'expérience, état de mouvement actuel et/ou antérieur du sujet d'expérience, mode de vie du sujet d'expérience, en particulier l'activité professionnelle, les habitudes alimentaires, la fréquence de l'activité sportive de celui-ci.

7. Procédé selon l'une quelconque des revendications précédentes, dans lequel les valeurs du paramètre de référence décrivent en outre au moins en partie les conditions marginales d'un environnement.

8. Procédé selon la revendication précédente, dans lequel le paramètre de référence comprend en outre un ou plusieurs de ce qui suit : moment de la journée, température ambiante, pression atmosphérique, humidité de l'air, saison.

9. Procédé selon l'une quelconque des revendications précédentes, dans lequel la délivrance délivre en outre au moins une des informations suivantes :
la valeur du paramètre de référence à laquelle le signal biologique de contrôle est associé ; la valeur du paramètre de référence à laquelle le signal biologique de comparaison sélectionné est associé ; le signal biologique de contrôle ; le signal biologique de comparaison ; un écart entre le signal biologique de contrôle et le signal biologique de comparaison ; un écart entre la valeur du paramètre de référence à laquelle le signal biologique de contrôle est associé, et la valeur du paramètre de référence à laquelle le signal biologique de comparaison sélectionné est associé.

10. Procédé selon l'une quelconque des revendications précédentes, dans lequel les signaux biologiques sont l'un des suivants :
courbes de pression artérielle, numérations globulaires, électrocardiogrammes, électroencéphalogrammes, électromyogrammes, électrorétinogrammes, enregistrements de la composition de gaz respiratoire, enregistrements de la composition d'excrétions.

11. Produit-programme informatique, qui est mis en œuvre sur un support lisible par ordinateur et avec un code de programme qui amène un dispositif selon la revendication 12 à mettre en œuvre les étapes du procédé défini dans une des revendications 1 à 10.

12. Système (60) pour la surveillance de signaux biologiques d'un sujet d'expérience, comprenant :
- au moins un capteur (62), en particulier des électrodes ECG, pour la mesure d'un signal biologique de contrôle, en particulier d'un électrocardiogramme de contrôle, du sujet d'expérience ;
- une mémoire (64) pour la mise en œuvre de plusieurs signaux biologiques de référence, en particulier des électrocardiogrammes de référence, du sujet d'expérience, dans lequel une valeur respective d'un paramètre de référence est associée à chacun des plusieurs signaux biologiques de référence, dans lequel les valeurs du paramètre de référence décrivent au moins en partie des états physiologiques du sujet d'expérience et dans lequel les plusieurs signaux biologiques de référence se présentent en continu comme plusieurs intervalles d'une série chronologique ;
- une commande (66), conçue
∘ pour déterminer une valeur du paramètre de référence associée au signal biologique de contrôle, dans lequel le paramètre de référence comprend une fréquence cardiaque,
∘ pour sélectionner au moins un signal biologique de comparaison, en particulier au moins un électrocardiogramme de comparaison, à partir des plusieurs signaux biologiques de référence mis en oeuvre, sur la base de la valeur du paramètre de référence déterminée et des plusieurs valeurs du paramètre de référence qui sont associées aux plusieurs signaux biologiques de référence,
∘ pour comparer le signal biologique de contrôle et le signal biologique de comparaison, et
∘ pour délivrer une recommandation d'action sur la base d'un écart entre le signal biologique de contrôle et le signal biologique de comparaison.
